# EUROPEAN PATENT SPECIFICATION

(11) **EP 0 322 254 B1**
(45) Date of publication and mention of the grant of the patent: **02.03.1994**
(21) Application number: 88312311.9
(22) Date of filing: 23.12.1988
(51) Int. Cl.: C12N 5/12, C12P 21/08, A61K 39/395, C12N 15/00, C12P 21/00

(54) **Monoclonal antibody capable of suppressing T lymphocyte proliferation**
Monoklonaler Antikörper fähig zum Unterdrücken der Proliferation von T-Lymphozyten
Anticorps monoclonal capable de supprimer la prolifération des lymphocytes-T

(30) Priority: 23.12.1987 US 137547; 12.12.1988 US 283339
(43) Date of publication of application: 28.06.1989
(73) Proprietor: Becton, Dickinson and Company, Franklin Lakes, New Jersey 07417-1880 (US)
(72) Inventor: Maino, Vernon, Los Altos, California (US); Buck David W., El Granada, California (US)
(74) Representative: Ruffles, Graham Keith

(56) References cited:
- EP-A- 0 097 518
- THE JOURNAL OF IMMUNOLOGY, vol. 137, no. 10, 15th November 1986, The American Association of Immunologists, US; V.C. MAINO et al.:

## Description

### Field of the Invention

The present invention relates to the production and use of a monoclonal antibody capable of suppressing cell-mediated immunity, and more specifically, to a monoclonal antibody capable of suppressing T lymphocyte proliferation induced as a result of antigenic or mitogenic stimulation.

### Background of the Invention

A hallmark feature of cell-mediated immunity, and one that has been exploited for in vitro measurement of T cell function, is the proliferation or mitotic response of T lymphocytes to specific antigens, allo-antigens or polyclonal mitogens.

Without adopting any specific theory, it is generally believed that T cell proliferation is a two step process involving a recognitive event followed by an activation event. The first or recognitive event (also called "competence") is believed to result from the binding of a ligand (e.g., antigen or mitogen) to the T cell receptor complex (i.e., α/B chains [WT31], CD3 complex [Leu-4] or CD2 antigen [Leu-5b]). Thus, T lymphocytes can be primed for activation by mitogenic lectins (e.g., Phytohemagglutinin [PHA] or Concanavalin-A [Con-A]) or monoclonal antibodies (e.g., Anti-Leu-4 or two or more anti-CD2 antibodies).

Once the ligand is bound, the cell exits the G₀ phase. The recognitive event then is believed to trigger the secretion of lymphokines, including Interleukin-2 (or "IL-2"), primarily by the helper subset of T lymphocytes. In addition, this event is believed to initiate the T cell surface expression of the IL-2 receptor complex.

The second or proliferation step (also called "progression") results from the binding of IL-2 to its high affinity receptor. Once bound to the receptor, a transduction event occurs resulting in gene activation which subsequently causes the cell to enter the S phase and proliferate.

The IL-2 high affinity receptor has been proposed to be a complex of at least a 55-60kD (or "p55") glycoprotein and a 70-75kD (or "p70") protein expressed on the cell surface of activated lymphocytes. Both of these structures apparently are capable of binding the IL-2 lymphokine; however, it is only when the two proteins exist together as a complex do they bind IL-2 with high affinity, and it is only binding to the high affinity receptor complex that results in T cell activation. More recently, it has been suggested that a third chain may be involved directly or indirectly in the formation or operation of the high affinity receptor.

A number of antibodies now exist which recognize the p55 glycoprotein or CD25 antigen (e.g., anti-TAC, 7G7/B6 or Anti-IL-2R); however, there confirmed reports of antibodies which recognize the p70 structure. Anti-CD25 antibodies show partial blocking of IL-2 dependent T cell proliferation, although this occurs only when IL-2 is limiting. Thus, in situations that more closely resemble antigen stimulation, such as mitogenic stimulation (e.g., with PHA), antibodies such as Anti-IL-2R are generally ineffective at blocking T cell proliferation, and thus achieving immunosuppression. similarly, monoclonal antibodies (or "MAbs") directed against cell surface structures partially involved in cell activation (e.g., Anti-Leu-5b) do not suppress T cell proliferation when the cells are subsequently exposed to IL-2. Further, they only partially suppress PHA activation. As a result, none of the antibodies currently available can block or suppress IL-2 directed cellular immunity.

The failure of these antibodies to block cell-mediated immunity has several consequences. In organ transplantation, for example, the grafting of the non-self organ will act as the recognitive step. Without means to block proliferation, rejection may occur. Conversely, in bone marrow transplantation, the lethal irradiation of the recipient's hematopoietic system and subsequent bone marrow transplant could lead to the rejection of the host's tissues by the graft resulting in graft versus host disease. Again, by blocking proliferation by providing the recipient with an effective amount of a blocking agent, GVHD may be prevented or at least its symptoms ameliorated.

Alternatively, an antibody which blocked cell proliferation could be used diagnostically to monitor IL-2 responsive lymphocytes or to monitor cell-mediated inflammation. Such diagnostic and therapeutic applications will only be effective if the monoclonal antibody will block the proliferation step.

Two T cell specific activation antigens, designated L-35 and L-36, are described in J. Immunology 137 3093 (1986). These antigens recognise a 97kd structure on activated T cells, but do not block proliferation of T cells stimulated by mitogens or a tumor cell alloantigen.

### Brief Description of the Invention

The present invention provides a hybridoma deposited under the accession number ATCC HB-9576, and the monoclonal antibody produced by this hybridoma, which antibody has our designation L129 (clone 8F5).

More generally, the present invention provides a monoclonal antibody having substantially the same cellular reactivity as a monoclonal antibody produced by the hybridoma deposited under the accession number ATCC HB-9576.

The monoclonal antibody L129 (clone 8F5) has unique functional properties in that it effectively blocks T cell proliferation and thus acts as a blocking agent. L129 is an IgG₂ₐₖ antibody with a molecular specificity for a 95kD structure expressed on mitogen activated T lymphocytes. The antigen recognized by L129 is weakly expressed on normal peripheral blood mononuclear cells (or "PBMC"), but exhibits increased expression following activation. It appears unique in its ability to recognize a 95kD structure and block cell proliferation. Without being bound to any specific theory, L129 may block proliferation by affecting the IL-2 high affinity receptor complex in some manner. Whether the 95kD protein is part of the high affinity receptor and the affect is on the binding of IL-2 to the receptor or is the result of a cascade of events brought about by the binding of the MAb to a 95kD structure, L129 blocks activated T cell proliferation.

The monoclonal antibody is the result of the fusion of murine drug marked plasmacytoma cells (or "fusion partner", e.g., SP2/0 Ag 14) with cells from the spleen of a mouse immunized against PHA-activated T lymphoblasts. Hybridoma supernatants from this fusion were screened for reactivity with PHA blasts. The 8F5 clone ultimately was selected.

The monoclonal antibody produced by this clone can be recovered from the supernatant and purified. Alternatively, it can be injected into a mouse strain and purified from ascites. As a reagent, it can be labeled with a fluorescent molecule or enzyme, or indirectly detected by use of a second antibody or similar molecule which has been labeled. By immunoabsorbant assay (ELISA), the presence of the bound monoclonal antibody can be detected.

It will be appreciated that other means may be used to produce similar antibodies. For example, other cells bearing the 95kD protein may be used as immunogens, and other species or cell lines may be used for immunization or fusion partners respectively. Alternativelyl hybridomas may be formed using Epstein Barr virus for transformation of cell lines. Finally, for therapeutic purposes, L129 may be made chimeric or mosaic by the methods disclosed in Morrison et al., EP 85 305 604.2, published 8 July 1985, or Winter, UK Pat. Appl. GB 2188638 filed March 26, 1987, respectively.

### Brief Description of the Drawings

Fig. 1. is a plot of MAb concentration versus ³H-thymidine incorporation for PHA activated T cells;
Fig. 2 is a plot of MAb concentration versus ³H-thymidine incorporation for anti-CD3 activated T cells;
Fig. 3 is a plot of MAb concentration versus ³H-thymidine incorporation for anti-CD3 activated T cells in the presence of 0.5% IL-2;
Fig. 4 is a plot of MAb concentration versus ³H-thymidine incorporation for IL-2 activated T cells;
Fig. 5 is a plot of time of L129 addition to PHA activated T cells versus ³H-thymidine incorporation;
Fig. 6 is a histogram of fluorescence for T cells labeled with goat anti-mouse Ig-FITC after exposure to an irrelevant IgG₂ₐₖ MAb control and T cells labeled with goat anti-mouse IgG-FITC after exposure to 1 ug of L129;
Fig. 7 is a histogram of fluorescence for T cells labeled with goat anti-mouse Ig-FITC after exposure to various anti-CD25 MAbs and L129; and

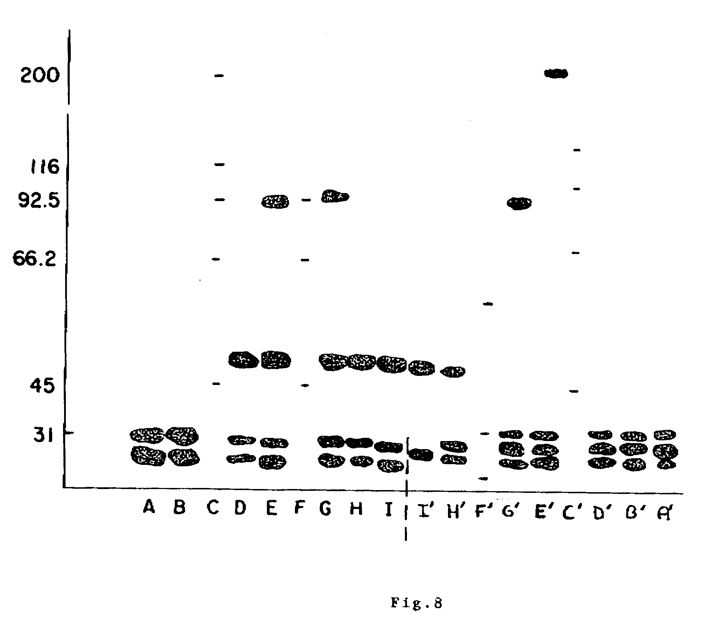

Fig. 8 is a representation of a 10% SDS-PAGE gel under reducing and non-reducing conditions (indicated by prime symbol) showing the molecular specificities of anitgens immunoprecipitated by L129 and other antibodies from detergent lysates of activated T cells wherein A is 2C10 (non-commercial, IgG₂ₐ directed against activation antigen on T cells), B is 1G3 (non-commercial, directed against p55 subunit), C is a high molecular weight standard, D is Anti-H-2K^{k}, E is Anti-Transferrin Receptor, F is a low molecular weight standard, G is L129, H is L54 and I is 4D10 (non-commercial, directed against p55 subunit).

The "CD" designation is the international standard for the "cluster designation" antigens to which monoclonal antibodies have been prepared. Unless noted, all monoclonal antibodies described herein, except L129, are commercially available from Becton Dickinson Immunocytometry Systems.

### Detailed Description of the Invention

Isolation of the 8F5 clone was as follows. Balb/c mice were immunized once I/P with 10⁷ PHA-activated PBMC. PBMC were isolated from human blood by density dependent centrifugation on Ficoll-Hypaque (Pharmacia). The mice were boosted with three successive I/V immunizations of 10⁷, 5X10⁶ and 10⁷ PHA-activated T lymphocyte blasts (or PHA-activated PBMC) on days 20, 42 and 65 respectively. The mice were sacrificed on day 68 and spleens excised.

Spleen cells from the immunized mice were then fused with the continuous plasmacytoma fusion partner cell line SP2/0 Ag 14, M. Shulman et al., Nature, 276:269 (1978), in 35% polyethylene glycol. Similar procedures for preparations of spleen cells and fusion have been previously described in U.S. Pat. Nos. 4,172,124 and 4,196,265. It will be appreciated that the mouse-mouse fusion performed may be done with other rodent species (e.g., rat-rat). The isolation of L129, therefore, is not limited to mouse species.

The cells from the fusion were plated in 96 well microculture plates (B-D Falcon) in Dulbecco's Modified Eagle's Medium (GIBCO) containing 20% fetal calf serum ("FCS¨) and 15mM Hepes buffer (GIBCO). Azaserine-hypoxanthine (2ug/ml and 10⁻⁴M final concentration) was included as a selection media in order to select for hybrid growth. Incubation continued for 7-10 days or until visible colonies of hybrids appeared.

Supernatants from those wells in which cells grew were screened initially by Pandex immunoassay (Pandex Laboratories, Inc.) using PHA-activated T cells as the positive selection and an LB B lymphoid cell line as the negative selection. 5x10⁵ PHA-activated PBMC or 5x10⁵ LB B lymphoid cells were added to each well of a 96 well Pandex plate. To each well was added 25-50ul of supernatant. After 30 minutes, cells were washed in 0.15M phosphate buffered saline (PBS). Goat anti-mouse Ig antibody coupled to fluorescein isothiocyanate (FITC) then was added to each well. The results from the immunoassay were read using Pandex instrumentation.

Cells from those wells whose supernatant tested positive were replated in 24 well microculture plates and grown without selection media. Supernatants from each of these wells were then rescreened by adding supernatant to wells containing either activated T cells or LB cells as previously described. Goat anti-mouse Ig-FITC antibody again was added to each well. The cells from each of these wells then were subjected to analysis by flow cytometry on a FACS® 440 flow cytometer (Becton Dickinson Immunocytometry Systems).

Initially, the stained cells were examined for forward and side light scatter. A gate then was set for lymphocyte populations. The stained cells then were analyzed for fluorescence. From this analysis, clone 8F5 was isolated from the positive wells and subsequently renamed L129. L129 reacts weakly with normal PBMC and strongly with activated T Lymphocytes. It does not appear to react with macrophages and monocytes. L129 has been deposited with American Type Culture Collection, Rockville, Maryland under the terms of the Budapest Treaty, and has been given accession number HB-9576.

Further characterization of the L129 antibody was performed as follows. Initially, immunoprecipitates of detergent lysates were prepared from activated T lymphocytes labeled with ³⁵S-methionine in accordance with procedures known by those skilled in the art. The immunoprecipitates then were analyzed by 10% SDS/PAGE, and revealed that the L129 recognized a protein of 95kD on activated T cells. See Fig. 8.

L129 was further characterized by its functional ability. Generally, 1x10⁵ PBMC isolated from normal human donors were incubated in 200 ul of RPMI 1640 (GIBCO) plus 10% FCS in a microtitre plate in the presence of varying concentrations of MAbs including relevant and irrelevant controls. Percent immunosuppression was measured as the percent decrease in the uptake of ³H-thymidine as compared to the isotype control following three days of activation by the appropriate mitogenic ligand. To measure DNA synthesis, the cells were exposed to ³H-thymidine (0.4 uCi, New England Nuclear) for 18 hours prior to harvesting. Incorporation was measured by liquid scintillation counting.

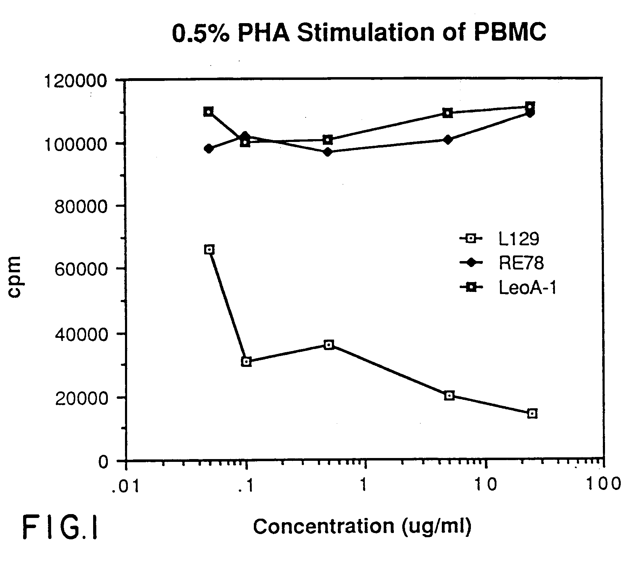

Referring to Fig. 1, PHA (0.5%) was used as the mitogenic ligand. PBMC were grown as above in the presence of L129, RE78 (a non-commercial anti-CD25 MAb [IgG₂ₐ] that recognizes the same epitope as the 2A3 clone) or Leo A-1 MAb (available from T cell Sciences, which recognizes a p70 activation structure) as a control. At levels of 25 ug/ml, maximum suppression of cell proliferation as measured by ³H-thymidine incorporation by L129 is achieved. In contrast, at levels of up to 50 ug/ml of Leo A-1 and RE78, however, no significant suppression of cell proliferation occurred.

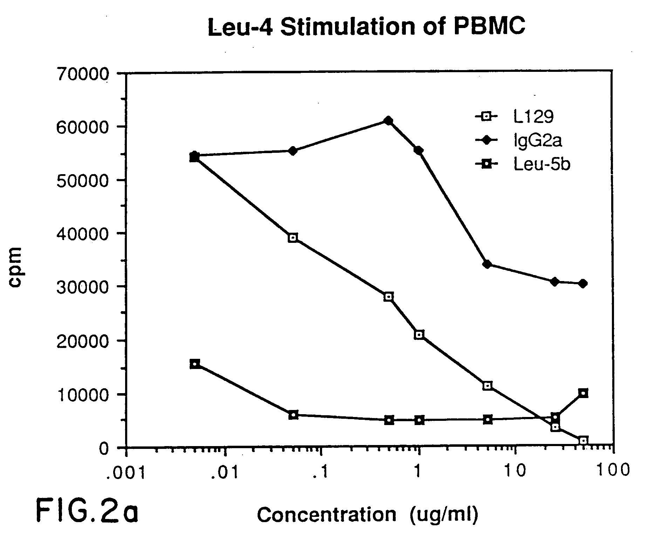

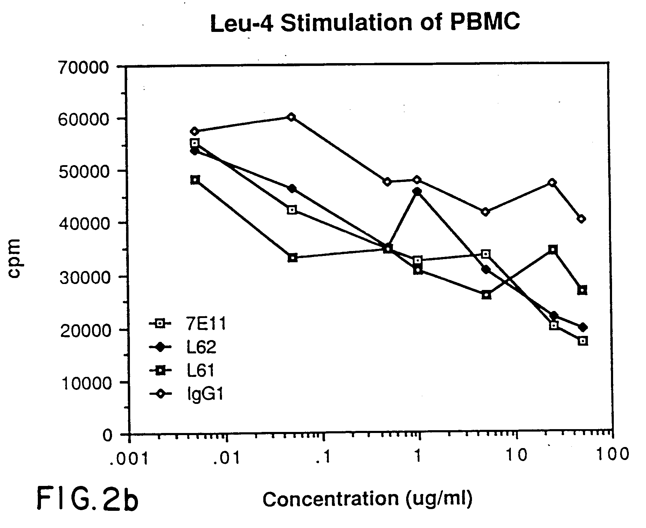

Referring to Fig. 2, Anti-Leu-4 was used as the mitogenic ligand. In Fig. 2b, anti-CD25 MAbs (7E11, L61 and L62 which are not commercially available) were used and IgG₁ was used as an irrelevant control. In Fig. 2a, H-2K^{k} (an irrelevant IgG₂ₐ control) was used as was Anti-Leu-5b. At high concentrations of anti-CD25 MAbs, some modest inhibition was observed, whereas, L129 gave stronger inhibition. Anti-Leu-5b also showed blocking ability but this MAb is believed to block competence, and thus, does not work at the proliferation step.

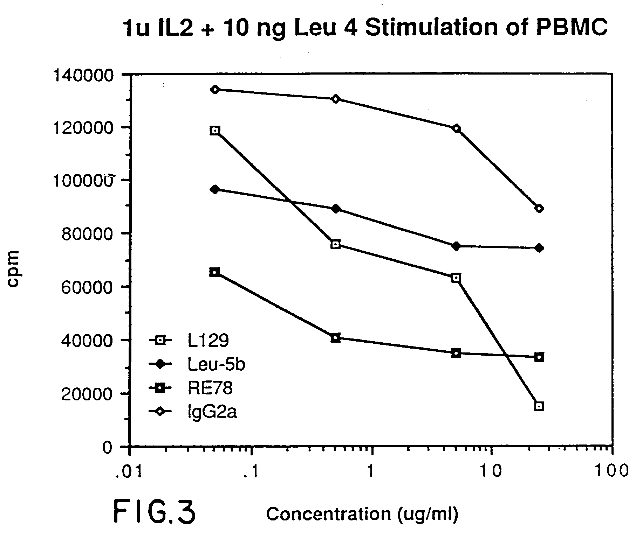

In contrast to the results obtained when Anti-Leu-4 is used as a mitogenic ligand alone, some anti-CD25 MAbs are considerably more effective in blocking proliferation as measured by ³H-thymidine incorporation when exogenous IL-2 is added to the activated cells. Referring to Fig. 3, RE78, like L129, will block proliferation of Anti-Leu-4 activated T cells when exogenous IL-2 is present.

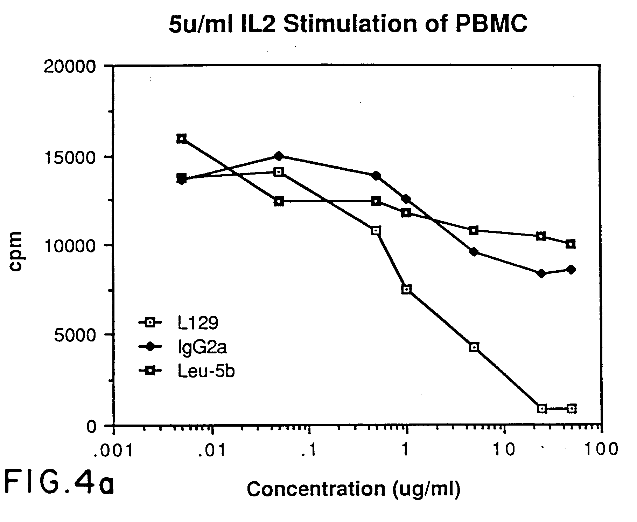

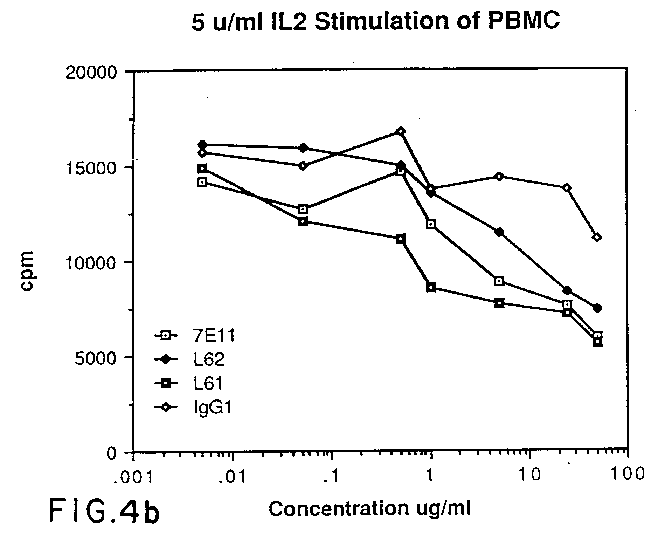

Not all anti-CD25 MAbs, however, will show this effect. Referring to Fig. 4, 1x10⁵ PBMC were exposed to 5 units/ml of IL-2 (Electronucleonics) which is a limiting amount in the absence of other stimulants. All other parameters including MAbs were as set forth in Fig. 2. As shown, L129 blocks proliferation of T cells at relatively low levels (i.e., 1.0 ug/ml), while the particular anti-CD25 MAbs used here show little to no blocking effect.

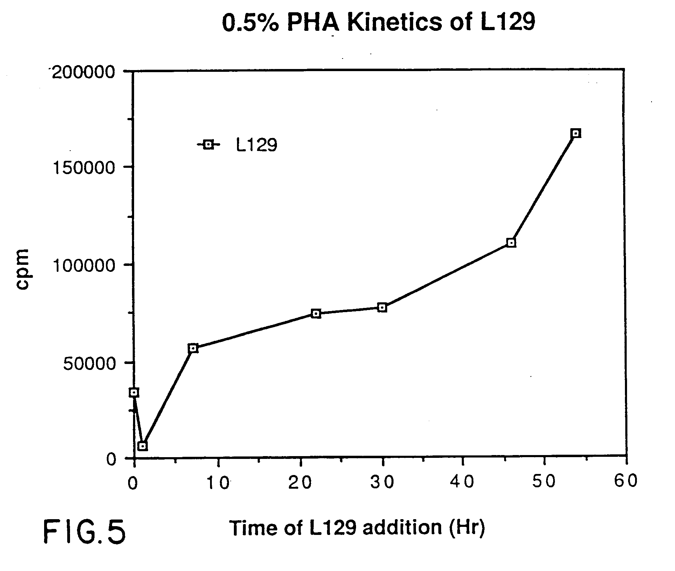

Finally, L129 appears to be most effective when added early during activation. Referring to Fig. 5, 25 ug of L129 were added to PHA activated T cells at various times after activation. Although the activated cells were not synchronized, L129 is more effective when added between 0-6 hours after addition of PHA than if added at later times, as measured by ³H-thymidine incorporation. It has been further shown that this result is not due to the blocking of the release of IL-2 by the activated cells. In contrast, cells activated with PHA when exposed to Anti-Leu-5b do show inhibition of IL-2 release.

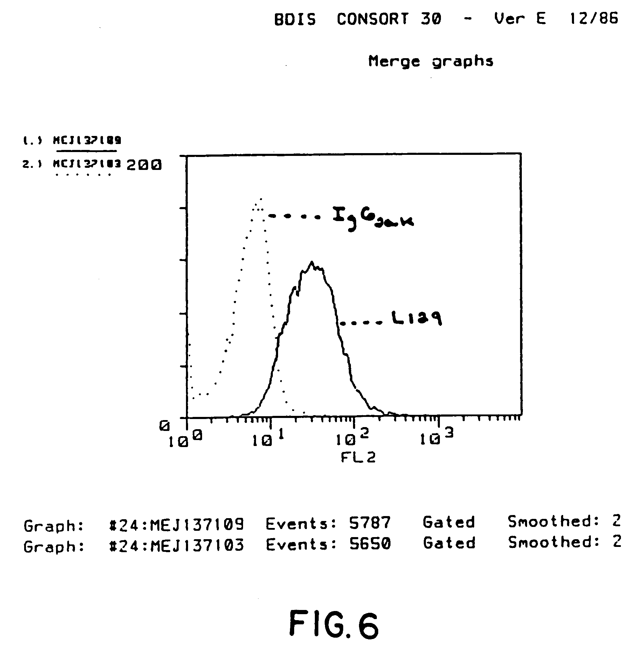

Apart from these functional assays, further flow cytometry analyses were performed. Referring to Fig. 6, PHA activated PBMC were plated as above and varying amounts of L129 were added. An irrelevant IgG₂ₐₖ MAb was used as a control. Goat anti-mouse Ig-FITC antibody then was added. Cells were then washed and screened using a FACScan^{Tm} flow cytometer after setting a lymphocyte gate by forward and side light scatter. Fig. 6 demonstrates the background fluorescence for cells stained with the IgG₂ₐₖ control and cells stained with L129.

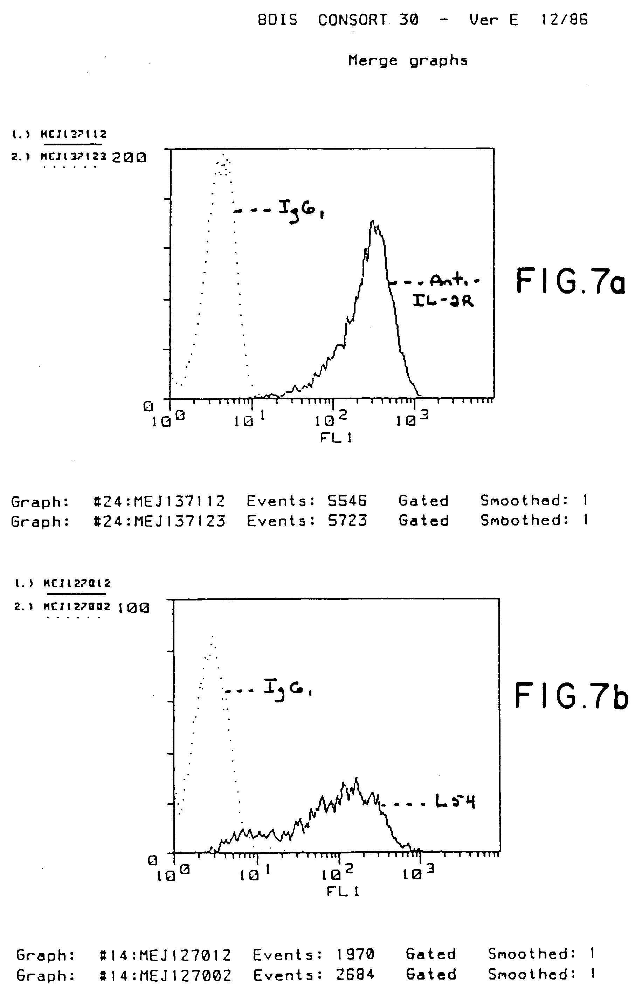

Referring to Figs. 7a and b, anti-CD25 MAbs, Anti-IL-2R and L54 (a non-commercial, IgG₁ isotype) were used as in Fig. 6 and compared with L129 in Fig. 6. Again, IgG₁ was used to show background fluorescence. Flow cytometric analyses indicate a total population shift as detected by L129 staining in Fig. 6. The bimodal staining seen with the anti-CD25 MAbs in Figs. 7a and 7b suggests that L129 does not recognize CD25.

Both the functional and flow cytometry analyses, therefore, are consistent with the finding that L129 causes immunosuppression apparently by blocking the proliferation of T lymphocytes through a receptor that it is not related to the p55 IL-2 molecule. As a result, L129 is a useful MAb in those research, diagnostic and therapeutic applications that require immunosuppression, and may be used accordingly.

## Claims

1. A hybridoma deposited under the accession number ATCC HB-9576.

2. A monoclonal antibody having substantially the same cellular reactivity as a monoclonal antibody produced by the hybridoma deposited under the accession number ATCC HB-9576.

## Patentansprüche

1. Hybridom, das unter der Eingangsnummer ATCC HB-9576 hinterlegt wurde.

2. Monoklonaler Antikörper mit im wesentlichen derselben zellularen Reaktivität wie ein monoklonaler Antikörper, der durch das Hybridom produziert wird, das unter der Eingangsnummer ATCC HB-9576 hinterlegt wurde.

## Revendications

1. Hybridome déposé sous le numéro de référence ATCC-HB 9576.

2. Anticorps monoclonal ayant substantiellement la même réactivité cellulaire qu'un anticorps monoclonal produit par l'hybridome déposé sous le numéro de référence ATCC-HB 9576.
